# EUROPEAN PATENT APPLICATION

(11) **EP 4 712 014 A1**
(43) Date of publication of application: **18.03.2026**
(21) Application number: 24805998.2
(22) Date of filing: 09.05.2024
(51) Int. Cl.: G06Q 50/20, G09B 7/02, A61B 3/11

(54) **SYSTEM AND METHOD FOR OBTAINING AND DOCUMENTING LEARNING METRICS, AND EQUIPMENT, SYSTEM AND METHOD FOR OBTAINING BIOPHYSICAL SIGNALS AND ASSISTING LEARNING DEVELOPMENT**

(30) Priority: 12.05.2023 BR 102023009160; 14.09.2023 BR 102023018618
(71) Applicant: Learnomics Consultoria Educacional Ltda, 01411-010 São Paulo (BR)
(72) Inventor: REMER, Ricardo Amaral, 01411-010 São Paulo (BR); AGUIAR, Simone Villaça, 04509-000 São Paulo (BR); ALVARENGA, Linda Omar Bernardes De, 04135-001 São Paulo (BR)
(74) Representative: Pace Napoleone, Maria
(86) International application number: PCT/BR2024/050192
(87) International publication number: WO 2024/234070

(57) **Abstract**

The present invention is in the field of pedagogy and systems for supporting and documenting learning, as well as organizing and certifying educational journeys and lifelong learning. More specifically, the invention describes a system and a method for obtaining and documenting learning and learning development metrics of individuals, and a digital learning repository, to which certification seals and a digital wallet with reward metrics or ESG metrics can be incorporated. The invention also provides a non-invasive method for capturing physiological, metabolic or physical signals related to the individual's cognitive effort; processing said signals and generating metrics or indices therefrom; and a feedback system that uses said signals, metrics or indices as a criterion for selecting learning contents to be made available to the individual. The system and method of the invention are particularly useful for: providing reliable learning indicators; improving the process of evaluating teaching programs and/or promoting the improvement of the learning performance of individuals, even when in collective teaching programs; and providing a digital learning repository or Digital Learning Wallet that integrates all of the individual's academic information in an easily accessible, secure and auditable location.

## Description

### Field of Invention

The present invention is in the field of pedagogy and systems for supporting and documenting learning, as well as organizing and certifying educational journeys and lifelong learning. More specifically, the invention describes a system and a method for obtaining and documenting learning metrics of individuals and learning development metrics of individuals, and a digital learning repository, to which certification seals and a digital wallet with reward metrics or ESG metrics can be incorporated. The invention also provides a non-invasive method for capturing physiological, metabolic or physical signals related to the individual's cognitive effort; processing said signals and generating metrics or indices therefrom; and a feedback system that uses said signals, metrics or indices as a criterion for selecting learning contents to be made available to the individual. Optionally, the invention further comprises a tool for providing suggestions for teaching practices specific to the individual's needs. In one embodiment, the system of the invention comprises a tool for building databases of learners' profiles and learning metrics. In one embodiment, the system comprises a tool for tokenizing learning performance metrics and rewarding meritocracy. The system and method of the invention are particularly useful for: providing reliable learning indicators; improving the process of evaluating teaching programs and/or promoting the improvement of the learning performance of individuals, even when in collective teaching programs; and providing a digital learning repository or Digital Learning Wallet that integrates all of the individual's academic information in an easily accessible, secure and auditable location.

### Background of the Invention

Human intellectual development is a field of ongoing interest. On the one hand, there is a vast amount of literature involving teaching processes and programs, that is, predominantly focused on contents and ways of passing it on. However, the prior art lacks tools for (1) concentrating diplomas and educational or professional experiences in a single virtual address and in a certified manner in Blockchain or other auditable certification and (2) generating, evaluating and documenting effective learning metrics, that is, from the individual perspective of each learner. More specifically, the prior art lacks tools that are simultaneously: (i) objective; (ii) free from the influence of the analysis subjectivity of people/teachers/pedagogues; (iii) interactive; (iv) that produce a knowledge base of learning metrics and the overcoming such difficulties; (v) authenticated documentation of learning metrics and academic history; (vi) a digital repository containing this data, with simplified access. The present invention provides a solution to these problems, in addition to providing a tool for the study of patterns derived from the analysis of the knowledge base.

There are various technological approaches to assist the cognitive development process of people with learning difficulties or cognitive deficits, including people with intellectual disabilities, learning disorders, Down syndrome, or those considered to be on the autism spectrum. In addition to being extremely limiting, known approaches have substantial gaps both in equipment and methods and the practices and technologies required for the cognitive development of these people.

One of the predominant factors in limiting the reach of such approaches is the application of pedagogical practices without a prior opinion that appreciates limitations and possibilities in a tailored and customized way, as this is ineffective in evaluating the previous state of individual knowledge, whether in the learning of people with some type of difficulty or disability or even in people without these limitations.

It is also desirable in the prior art to have a consistent approach to monitoring the progress and setbacks longitudinally, through performance indicators that qualitatively and quantitatively guide the cognitive development of the person in different operational contexts.

Specifically within the context of Intellectual Disability and issues related to cognitive deficits, several patents related to the analysis and treatment of these subjects have been filed.

On the other hand, some patents and patent applications found in searches mention the term "learning", but most documents refer to computer systems for machine learning, which are not related to the present invention - it is related to tools for generating, analyzing and documenting learning metrics of individuals (humans or animals). They will therefore not be described in this background section as they are not related to the present invention.

U.S. Patent Application US2004002888 (Accenture Global Services GMBH), entitled "Business Driven Learning Solution", was published on January 1, 2004. Said document discloses a method for managing an organization's learning and training needs, being responsive to the temporality of business dynamics, its goals and strategic objectives. The method employs interdependent and interrelated business process modules for identifying and prioritizing learning needs, selecting learning approaches, developing contents, coordinating and effectively delivering instructions, as well as the overall management and administration of learning efforts. The method further includes tracking and reporting various performance metrics that provide the allocation of learning efforts to provide cost-effective outsourcing of an organization's learning needs. This is not related to the present invention, as it does not address the issue of learning metrics, identification of difficulties or adjustments of cognitive load from the student or educators viewpoint.

U.S. Patent Application US2014344177 (Monk Akarshala Inc), entitled "Learner Ranking Method in a Modular Learning System," was published on November 20, 2014. This document discloses an equipment and a method for ranking learners in a modular learning system. The system includes metadata used to define performance metrics, which in turn are used in performance assessments of each learner in each learning module. The modular system provides the designation of the performance ranking of a group of learners. This is not related to the present invention, as it does not address the issue of learning metrics, customized identification of difficulties or adjustments of cognitive load from the student or educators viewpoint.

U.S. Patent Application US2016364996 (Learning Threads Corp), entitled "Matching Learning Contents to Learners," was published on December 15, 2016. Said document discloses a system that provides relationships between learning contents items in a network. In said system, information about learning contents is generated from the analysis of the learning contents, and graphs showing the relationships between these contents are generated. Statistical analyses of the learners' characteristics are used to provide learning metrics, which are used to customize learning contents to the learners. This is not related to the present invention, as it does not address the issue of learning metrics, identification of difficulties or adjustments of cognitive load from the student or educators viewpoint.

U.S. Patent Application US2017256172 (Civitas Learning Inc), entitled "Student Data-to-insight-to-action-to-learning Analytics System and Method," was published on July 9, 2017. Said document discloses a system that uses an evidence-based active knowledge database. The system computes predictions of the students' success and engagement, as well as predicted impact and interventions. Said database is updated by means of a multidimensional analysis of the interventions impact. Multidimensional analysis includes the use of changes in KPIs (key performance indicators) applied to pilot-students after each intervention is administered, and the dynamic matching of the pilot-students exposed to appropriate interventions to other students who were not exposed to the appropriate interventions. This is not related to the present invention, as it does not address the issue of learning metrics, identification of difficulties or adjustments of cognitive load from the student or educators viewpoint.

Chinese patent application CN 201710208847.2, entitled (in a free translation into English) "Treatment System for Attention-Deficit/Hyperactivity Disorder in Children", was published on July 14, 2017. This document refers to a treatment device and a treatment system. The first consists of an assessment module, which aims to identify the attention-deficit and the severity of the symptoms in the subject, and the second provides training means for treating the plurality of essential domains in executive function that are compromised. Said document is based on the individual's ontogenetic characteristics, focusing on what the individual brings with them in terms of skills and abilities, limitations and commitments. Emphasis is given on the retrospective nature of skills (what he/she knows or does not know how to do) and not on the prospective nature, that is, what the person with a disability can develop with the intervention of the social other. Said document does not solve the same problems as the present invention.

Patent application BR102016026833-8 was filed on June 5, 2018 and is entitled "Sistema de Neurometria para a Análise e Treinamento Funcional do Sistema Nervoso e Cognitivo" [Neurometry System for the Analysis and Functional Training of the Nervous and Cognitive System]. Said document discloses an approach that involves the use of sensors connected to the individual's head and a software that processes the received signals. Said system performs a functional neurometry analysis to evaluate the performance of the nervous and cognitive system. The data is sent to the software that filters, analyzes, calculates and finalizes, generating graphical, numerical and written results specific and exclusive to the application of neurometry. This same software can be used for specific functional training in neurometry for anxiety control, inductive sleep physiology, emotional variability, functional cardiac coherence, functional cardiac variability, functional respiratory amplitude and frequency, functional respiratory capacity, functional physiological response, computerized progressive muscle relaxation, functional monitoring, computerized cognitive adjustment, functional analysis of the nervous and cognitive system, functional fitness analysis, DLO analysis (positional maneuver) and functional analysis of brain wave predominance. Said document relates to the use of sensors to obtain physical data from people, which is not related to the present invention.

U.S. Patent Application US2018350015 (Linkedln Corp), entitled "e-Learning Engagement Scoring," was published on June 12, 2018. Said document discloses objective metrics and metrics analysis to quantify a user's engagement in a digital learning system. One metric is a single-number summary that describes the learner's use of the product. Said single-number summary is produced from the weighted and normalized sum of individual metrics. Metrics may include, for example, system activation, login, viewing, and other usage indicators with part of said weighted sum, which is normalized by comparison with the metrics of other users exposed to the same or similar contents. This is not related to the present invention, as it does not address the issue of learning metrics, identification of difficulties or adjustments of cognitive load from the student or educators viewpoint.

U.S. Patent US 10276055 (Mattersight Corp), entitled "Essay Analytics System and Methods", was granted on April 30, 2019. Said document discloses a method and system for evaluating written essays and determining student attributes from their essays. The method includes the steps of: identifying keywords in the text/essay; associating one or more metrics of the respective student; providing a score to the essay for one or more metrics of the student using a psychological-behavioral model; and recommending teaching methods based on the score.

The international patent application WO 2019102493 (ESSM Innovative Teaching Concepts), entitled "Digital Teaching Management System", was published on May 31, 2019. This document discloses a method and system for managing the digital teaching of an academic curriculum. The method includes: determining, through a contents management tool, course contents related to topics from external sources based on the duration time provided for teaching the topics. The method includes the contents management tool modifying the contents according to the user's preference, user profile or previous user performance. The method also includes the generation, by the contents management tool, of notifications when the duration of the topics does not satisfy the predetermined teaching duration of each topic in a session. The method also includes the generation, by the contents management tool, of a report of the digital course plan when the duration of the topics meets the predetermined teaching duration of each topic in a session. This is not related to the present invention, as it does not address the issue of learning metrics, identification of difficulties or adjustments of cognitive load from the student or educators viewpoint.

Australian patent application AU2018316619, published on March 5, 2020 and entitled (in a free translation into English) "Cognitive Platform Including Computerized Elements", discloses an apparatus, system and method for generating a quantified indicator of cognitive abilities in an individual. In certain settings, said approach can be implemented to improve the individual's cognitive abilities. Said device generates a quantified indicator of cognitive skills in an individual and is based on the notion of cognitive skills (motor coordination, adaptation, psychomotricity and language), which can be trained through school intervention. However, this notion is linked to the idea of data processing to develop solely in the individual isolated from their contexts, stimuli for understanding, perception and integration into certain tasks. The assumptions underlying this model advocate a mechanistic conception of the human being, advocating that skills can be trained through action in the school environment. Another limitation of this approach is the fact that it is based on the ontogenetic dimension of the individual, forgetting that the cognitive, affective and social dimensions are constituted in and by the action of the social other. Not to be confused with the present invention.

U.S. Patent Application US 2021407310, entitled "Teaching and Learning Platform," was published on December 30, 2021. Said document discloses a teaching platform. The platform comprises: a hardware module containing an Administrator User Interface configured for an administrator user to operate a profile module comprising an administrator profile, a class profile; a teaching module comprising: a curriculum, a test module and a schedule; an Artificial Intelligence module configured to operate the Administrator User Interface when the administrator is absent; a student hardware module comprising a Student User Interface configured to operate a learning module containing a plurality of test subjects, a virtual tutor; and a communication module for communication between the student and the administrator. This is not related to the present invention, as it does not address the issue of learning metrics, identification of difficulties or adjustments of cognitive load from the student or educators viewpoint.

Patent application BR102022003708-6, entitled "SISTEMA E MÉTODO INTEGRADO PARA A EMISSÃO DE ATIVOS DIGITAIS OU TOKENS LASTREADOS EM MÉTRICAS DE CONSERVAÇÃO AUTENTICADAS" [INTEGRATED SYSTEM AND METHOD FOR ISSUING DIGITAL ASSETS OR TOKENS BACKED BY AUTHENTICATED CONSERVATION METRICS], was filed by one of the present inventors, among others, and was published on September 5, 2023. Said document discloses an integrated system and method for issuing digital assets or tokens backed by authenticated conservation metrics. The system is particularly useful for the measurement, documentation, authentication and tokenization, in blockchain platforms or technologies, distributed digital ledger, of metrics related to sustainable or environmental conservation attitudes and/or projects. The system comprises: one or more devices for measuring physical quantities associated with the conservation or recovery of environmental assets; and a distributed digital ledger process of authenticated metrics. In one embodiment, the system additionally comprises a device for verifying the authenticity of the hardware and software that process the measured signals and that make up the measuring device. Said system is particularly useful for the online documentation of authenticated metrics that serve as a collateral for the digital assets documented by the system itself, being a useful tool to boost investments in projects involving crypto assets, in addition to providing real, online and publicly auditable collateral for digital assets. This is not related to the present invention, as it does not address the issue of learning metrics, identification of difficulties or adjustments of cognitive load from the student or educators viewpoint. However, in one embodiment of the present invention, said system is used to document learning metrics.

Some approaches to using digital tools to support learning are available on specialized websites.

An example is the approach available at www.disciplina.io/whitepaper.pdf. The so-called "Disciplina" [Discipline] is a proposal to build a Blockchain with the purpose of creating verified profiles based on professional and academic achievements. Thus, "Disciplina" is a multifunctional blockchain for projects in the education and recruitment sectors, which provides transparency in work and creates conditions for maintaining the confidentiality and trust of information added by system participants. Said approach uses tokens that do not use any other blockchain and is being developed according to the demands of the educational and recruitment sectors, taking their specifications into account. However, it does not have a pedagogical vision, and its main focus is building databases for recruiters.

The MIT platform, entitled Open Source Student Wallet, is available at:https://digitalcredentials.mit.edu/docs/Open%20Source%20Student%20Wallet%20Final %20Report%20-%20Public%20Web%20Version.pdf). This approach was developed in 2020, based on an agreement between MIT and the US Department of Education to design and implement, as an open source, a digital wallet for controlling educational credentials. Said project is related to the W3C - World Wide Web Consortium and then the Learner Credential Wallet (https://openlearning.mit.edu/news/developing-open-source-wallet-digital-credentials).

Another dimension related to the present invention is the learning data proxies documentation obtained through biophysical signals by using equipment, which are also documented within the scope of the present invention. In this context, some close but not anticipatory antecedents are described below.

The article entitled *"*Pupillary Responses for Cognitive Load Measurement to Classify Difficulty Levels in an Educational Video Game: Empirical Study" was published in JMIR Serious Games. 2021 Jan-Mar; 9(1): e21620, published online on January 11, 2021, doi: 10.2196/21620. Said article discloses that a learning task perceived as either easy or difficult can cause unsatisfactory learning results, and that games data with errors, attempts, and time to complete a challenge are widely used to estimate the level of difficulty. The article shows that pupillometry is used to measure cognitive load (mental effort) and that it can therefore be used to describe the perceived difficulty of a task.

The article entitled *"*Pupil size as a window on neural substrates of cognition" was published in Trends Cogn Sci., with its manuscript available at PMC on June 1, 2021, being published in the edited version as: Trends Cogn Sci. 2020 Jun; 24(6): 466-480, published online April 21, 2020, doi: 10.1016/j.tics.2020.03.005. This article discloses that cognitively driven pupillary modulations reflect some underlying brain functions, and that, upon review, this is associated with the key role of three neural systems. First, cortical modulation of the pretectal olivary nucleus (PON), which controls the pupillary light reflex; second, the superior colliculus (SC), which mediates orientation responses, including pupillary shifts to salient stimuli; and third, the locus coeruleus (LC)-norepinephrine (NE) neuromodulatory system, which mediates the relationship between pupil-linked arousal and cognition. The article also discusses how these findings can help interpreting pupillary measurements in terms of activation of these three natural systems, including caveats, unanswered questions, and suggestions for future experiments to improve interpretations related to brain neurodynamics.

The article entitled *"*Individual pupil size changes as a robust indicator of cognitive familiarity differences", was published in PLOS ONE on January 21, 2022 (https://doi.org/10.1371/journal.pone.0262753). This article is based on the premise that cognitive psychology has been using pupillometry for a long time, but that its use has been very limited by interferences (such as luminance or colors) that make it susceptible to confusion. Particularly, that measuring pupil size requires sophisticated experimental designs to dissociate small changes in pupil diameter associated with cognitive processes from large changes in pupil diameter associated with changes in stimulus properties or the experimental environment. The article discloses a new pupillometry paradigm that adapts the pupil to the properties of the stimuli during a calibration or baseline period, in which the meanings of the stimuli or context are not disclosed, using an image mask (whose pixels are scrambled) wrapped around an intact image. Using this approach, the authors demonstrate that there is robustness between the context of pupillary responses and the degree of familiarity with products from well-known brands. The results show larger mean pupil diameter and larger peak pupil diameter for passively viewed images of familiar products, in addition to an extended posterior temporal component, representing differences in familiarity between participants (starting approximately 1400 ms after stimulus onset). These amplitude differences are present in almost all participants individually and vary between product categories. Interestingly, amplitude differences are absent in the calibration or baseline period, which suggests that involuntary pupil size measurement, combined with said paradigm, is useful for dissociating the cognitive effects of physical stimuli that cause interpretation confusion.

The article entitled *"*Pupil dilation reflects the time course of emotion recognition in human vocalizations"was published in Nature Scientific Reports volume 8, article number: 4871 (2018). In said article, considerations are made to the fact that processing emotional signals generally increases pupil diameter, and that this effect has been attributed to autonomous excitation driven by stimuli. And also, about the associations made in the literature between pupil diameter and decision-making during non-emotional perceptual tasks. The article discloses research results on the relationship between pupil size fluctuation and the process of emotion recognition. The experimental model used involved nonverbal vocalizations heard by participants (e.g., crying, laughing). The results showed that during emotion recognition, the pupillary response time was determined by the decision-making process and the maximum pupil diameter "betrayed" the emotion selection time. Pupillary responses showed the properties of decisions, such as perceived emotional valence and assessment confidence. Since, according to the authors, pupil dilation under isoluminance conditions is almost exclusively promoted by the release of norepinephrine (NE) in the locus coeruleus (LC), the results suggest an important role of the LC-NE system during emotion processing.

From what can be inferred from the literature researched, no documents were found anticipating or suggesting the teachings of the present invention.

### Summary of the Invention

The present invention solves several problems of the prior art and provides a system and a method to support obtaining and documenting learning metrics of individuals.

It is one of the objects of the invention to provide a tool to support obtaining learning metrics that is simultaneously: (i) objective; (ii) free from the influence of the analysis subjectivity of people/teachers/pedagogues; (iii) interactive; (iv) a tool for building a knowledge base of learning metrics and the overcoming such difficulties; (v) a tool for the authenticated documentation of learning metrics and academic history; (vi) a digital repository containing this data, with simplified access.

It is an object of the invention to provide a system and method comprising one or more of the following elements: (i) a learning analysis tool based on the comparison between prior knowledge and the knowledge acquired after pedagogical practice; (ii) a process for objective analysis of parameters abstracted from said analysis of an individual, thus generating metrics; (iii) a process for documenting the learning metrics of the individual through performance indicators or learning metrics; and (iv) a digital database or repository of learners' profiles, learning metrics and/or academic history, with simplified access.

In one embodiment, the system additionally comprises a tool to provide suggestions for pedagogical practices specific to the individual's needs. This tool is optionally gamified.

In one embodiment, the system additionally comprises a tool for tokenizing learning performance metrics.

In one embodiment, the learning metrics tokenization system is particularly useful as a tool for directly encouraging and rewarding the learner for each learning achievement.

One of the objects of the invention is to provide a tool to help understand the type of learning of students, which is essential to provide effective and customized education. Various methods and approaches have been used to assess a student's learning style, including questionnaires, observations, and performance analysis. However, these methods often have limitations in terms of accuracy and efficiency and can be time-consuming. In one embodiment, the invention provides a tool that employs artificial intelligence to accurately determine a student's learning style. In said embodiment, a learning type determination algorithm that uses artificial intelligence to identify the student's predominant learning style is used as a basis for the patterns and relationships obtained from data analysis, providing: (I) improved accuracy in identifying student learning type. (II) efficiency in data collection and processing through artificial intelligence techniques. (III) ability to provide highly customized teaching based on the type of learning identified. This results in more effective and customized education, enabling teaching to be adapted to the individual needs of each student. The application of artificial intelligence in this context has the potential to revolutionize the way we approach the learning process.

The system and method of the invention are particularly useful for: providing reliable learning indicators; improving the process of evaluating teaching programs and/or promoting the improvement of the learning performance of individuals, even when in collective teaching programs; promoting encouragement or reward for each learning achievement; and facilitating access to information/histories, through an easily accessible repository, with authenticated and tokenized information on learning metrics and academic history.

In one embodiment, the invention provides a system and a method for obtaining biophysical signals from the learner and for documenting learning metrics derived from said signals, forming a database of biophysical signals, of learning metrics derived from such signals. In one embodiment, the system and method of the invention uses said data or metrics as a criterion for selecting cognitive challenges or learning tasks for the individual.

The invention provides a non-invasive method for obtaining signals and documenting learning metrics from human beings and for selecting cognitive challenges or learning tasks for the individual, comprising:
- a non-invasive stage of capturing physiological, metabolic or physical signals related to the individual's cognitive effort;
- a stage of processing said signals and documenting them and/or generating metrics or indices therefrom; and
- a feedback stage that uses said signals, metrics or indices as a criterion for selecting learning contents to be made available to the individual.

In one embodiment, said method comprises:
- a stage of capturing physiological, metabolic or physical signals related to the individual's cognitive effort, selected from: eye tracking, pupilloscopy for measuring pupil diameter and/or the speed of pupil dilation or contraction, or combinations thereof;
- a step of storing, in a database, the measured data, and processing said data to generate metrics or indices therefrom, as well as storing, in the database, said metrics or indices obtained; and
- a feedback stage that uses said signals, metrics or indices as a criterion for selecting learning contents to be made available to the individual.

In one embodiment, this method is recursive and provides the generation of measurements, metrics and/or evolution indices of the learning process. In one embodiment, this method is used both individually and collectively, that is, the step of selecting cognitive stimuli or learning tasks to be presented can be done based on the average of the metrics or indices of a subgroup of individuals, with the equivalent contents being presented to everyone in the same subgroup instead of individually.

It is another object of the invention that a system of the invention comprises:
- a device for capturing physiological, metabolic or physical signals related to the individual's cognitive effort, selected from: an eye tracking device, a pupilloscope, a device that measures pupil diameter and/or pupil dilation or contraction speed, or combinations thereof, wherein said device sends signals to a processor;
- a processor for said signals that provides documentation thereof and/or the generation of metrics or indices therefrom, as well as the creation of a database in which such information is stored; and
- a feedback system that provides the selection of cognitive stimuli or learning tasks, from said database or other databases, according to pupillary measurements and/or metrics or indices derived therefrom, to be made available to the individual by a device that provides cognitive stimuli, whether visual, auditory, tactile, olfactory or combinations thereof.

In one embodiment, said system additionally comprises other devices selected from: a speaker or headset; a microphone; a screen for displaying images; a brightness or luminance meter in the eyes region; a device for generating brain images by magnetic resonance; an intracranial pressure meter; a polygraph; or combinations thereof. In this embodiment, the measurements and metrics associated with these measurements are also stored in the database.

In one embodiment, the system of the invention further comprises a system or tool for providing suggestions for pedagogical practices specific to the individual's needs. This tool is optionally gamified.

Another object of the invention is equipment to aid the development of learning. Said equipment comprises:
- a signal acquisition device selected from: an eye tracking device, a pupilloscope, a device that measures pupil diameter and/or pupil dilation or contraction speed, or combinations thereof;
- a processor for said signals to document them and/or to generate metrics or indices therefrom, and to create a database in which such information is stored; and
- a device for visual, auditory, tactile, olfactory feedback or combinations thereof, of cognitive stimuli or learning tasks, selected from the said database or from other databases according to pupillary measurements and/or metrics or indices derived therefrom.

These and other objects of the invention will be immediately appreciated by those skilled in the art and will be described in detail below.

### Brief Description of the Figures

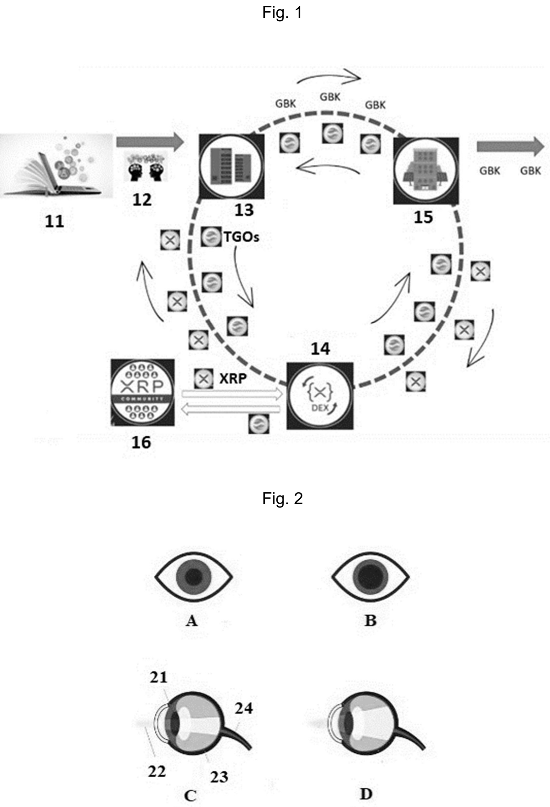

Fig. 1 shows a schematic representation of an embodiment of the invention in which the system (herein called LEARNOMICS) comprises a tool for tokenizing learning metrics. In 11, the platform is shown as exemplified in Example 1, producing the metrics indicated in 12; in 13, the token generation platform (TGOs) and certificates (GBKs) is shown; in 14, a centralized or decentralized crypto asset exchange is shown, which can operate commercially in conjunction with the crypto asset market (for example, Ripple's XRPs) or traders 16; in 15, one or more companies or people are shown that support the learning process through the purchase of crypto assets (TGOs) and exchange for certificates (GBKs).

Fig. 2 shows a schematic representation of a human eye, with the following indicated: in A, the eye in a normal state; in B, an eye with a dilated pupil; in C, an eye is shown in a schematic side view of the eye shown in A, with the following highlighted: 21 the pupil; 22 a beam of light; 23 the retina; and 24 the optic nerve; in D, an eye is shown in schematic side view of the eye shown in B, i.e., with the pupil dilated.

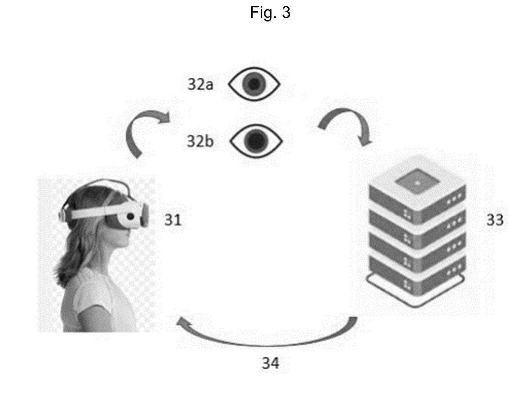

Fig. 3 shows a schematic diagram representation of an embodiment of a method and system of the invention, in which a pupillometry examination step results in the indication of the pupil diameter and/or the rate of change of the pupil diameter in response to a cognitive stimulus. A system for obtaining signals and documenting human learning metrics includes: a pupillometer 31 generating measurements of pupil diameter and/or the rate of change of pupil diameter 32a, 32b in response to a cognitive stimulus, which may be visual, auditory, tactile, olfactory or combinations thereof; the measurements performed by the pupillometer are stored in a database 33. Said database stores the actual measurements and/or metrics or indices derived from these measurements, as well as the cognitive stimulus presented to the individual, to provide a subsequent correlation between the stimulus and the measurements, metrics and indices.

### Detailed Description of the Invention

The present invention solves several problems in the prior art and provides a system and a method to support obtaining learning metrics from individuals.

The system and method of the invention are particularly useful for: providing reliable learning indicators; improving the process of evaluating teaching programs and/or promoting the improvement of the learning performance of individuals, even when in collective teaching programs; promoting encouragement or reward for each learning achievement; and facilitating access to information/histories, through an easily accessible repository, with authenticated and tokenized information on learning metrics and academic history.

The invention provides a tool to enable a shift in focus from teaching to learning processes. In this context, it is worth highlighting that the predominant mental model is TEACHING and not LEARNING. Therefore, current processes are focused on teaching and do not reflect learning or learning processes. This arises, at least in part, from the fact that the conventional structure of education systems is based on a minimum curriculum to be taught. The available metric, measured in tests, is the contents retention and not learning.

It is common for those who formulate the minimum curriculum to think that assigning something standardized to all students (who are not standardized) would be the best way. The false premise is that the teacher has the knowledge and the student does not, or that the teacher knows what is important to everyone. This outdated model of thinking derives from an old scenario, in which there was no bidirectionality (or multidirectionality) in the knowledge construction process. The problem is partly cultural and partly derived from a lack of technology or access to technology, which is gradually being overcome. The lack of knowledge or resistance, by educators, to the concept of innovation, particularly innovation in teaching/learning, also influences this context. The invention provides a path and a tool for these goals.

The invention anticipates trends in education, which include: hybrid teaching, e-learning, microlearning, socio-emotional education and customized teaching.

In one aspect, the invention provides a tool for organizing and managing studies according to an individual's learning goals.

The invention is based on the recognition that individuals/students have diverse abilities, learning paces and understanding styles - and that this is fundamental to the development of a more inclusive and effective learning system. The idea that all students should learn uniformly and at the same speed has been challenged for decades, and educators are looking for ways to better meet students' individual needs. Understanding that students have different ways of learning the same contents is an essential starting point for the invention tool, which provides truly customized education based on individual learning.

Each student brings a unique set of experiences, prior knowledge and personal characteristics. These individual differences are a challenge to conventional teaching approaches and are an opportunity in the present invention.

In "mass" teaching, individual nuances tend to be ignored, which can lead to the alienation of some students and a decrease in their motivation to learn, which directly impacts learning and the results of activities and assessments carried out by students.

In the present invention, a system is provided that allows the individual/student to learn at their own pace and how they prefer, with a customized approach. The customized approach considers differences in speed, learning styles, interests, and even individual limitations of each student. The invention's technology provides students with access to contents in different formats, participation in interactive activities, and assessments customized to their progress. This approach not only improves student motivation and engagement, but also strengthens their self-management and autonomy skills, valuable skills for today's ever-changing world. Furthermore, it recognizes and values the diversity of talents and ways of thinking. By providing an environment where students can learn at their own pace and how they prefer, the invention provides a useful tool for a more inclusive, customized and effective education that recognizes and values the uniqueness of each individual.

The system of the invention, in an aspect called DIGITAL LEARNING WALLET, comprises a platform that uses algorithms that consider the individual learning characteristics of each student. It is based on respect for individuality and the creation of strategies that take the interests of each student into account. It is an adaptive and intelligent, optionally tokenized, platform which uses software that proposes different activities for each student, adjusting these activities to suit their responses and reactions to the tasks. In such a platform, students have access to various learning experiences and rewards in the form of tokens, through, for example, games, videos, texts, exercises, group activities, receiving, in real time, feedback on their own performance.

In one embodiment, the invention employs artificial intelligence (AI) as a complementary tool. In education, AI enables the processing of large amounts of information about each student and the near real-time adaptation of contents and activities to meet their specific needs. In the present invention, AI is applied in various contexts, such as in the creation of virtual tutoring systems and in the development of customized contents, among others. Therefore, AI is a powerful tool to enhance customized education. By analyzing data on the student's performance, behavior, and learning preferences, AI systems offer valuable insights to educators, who can identify areas where a student is struggling and provide additional resources to help them overcome these obstacles. Additionally, AI can automatically adapt contents based on individual progress, keeping the learner engaged and challenged.

The invention provides, among others, the following technical effects:
*Student Knowledge:* The invention provides a way to get to know students well, understanding their abilities, learning styles, interests and specific needs. This requires ongoing assessment and an individualized approach to understanding how each student learns best.
*Curricular Flexibility:* The present invention provides for the delivery of a variety of contents options, allowing students to choose learning paths that align with their interests and goals.
*Diverse Teaching Methods:* The present invention, which has a customized approach, uses different teaching methods to accommodate diverse learning styles. This can include lectures, group discussions, individual projects, problem-based learning, among others.
*Student Self-Awareness to Self-Directed Learning:* The invention aims to encourage students to take more responsibility for their own learning. The system of the invention gives you the opportunity to set goals, manage your time, and make decisions about what and how to learn.
*Regular Feedback:* Continuous feedback is essential in the customized education provided by the present invention. This includes both feedback from the platform itself and the students' self-assessment. Feedback helps adjusting the learning process according to individual needs.
*Technology* as a *Tool:* The technology of the invention is useful for delivering customized contents, assessing student progress, and providing additional resources based on the student's performance.
*Collaboration and Interaction:* Despite the customized approach of the present invention, interaction between students and educators is still essential. Collaboration on projects, group discussions and sharing of ideas should encourage critical thinking and socialization.
*Flexible Assessment:* Customized assessments can involve a variety of formats, including tests, projects, presentations, and portfolios. The evaluation of the present invention focuses on understanding and application of knowledge, rather than just memorization.
*Individual Goals:* Setting individual learning goals helps students focus on their personal progress and achieve a sense of accomplishment, and the present invention helps the student view their personal goals and achieve them.
*Respect for Diversity:* Customized education values the diversity of students' abilities, backgrounds, and perspectives and provides more accurate metrics that take all of these aspects into account. The present invention promotes an inclusive environment where every student feels valued.

The invention may also be defined by the following clauses.

System and method for obtaining and documenting learning and learning development metrics of individuals comprising one or more of the following elements: (i) a tool to understand the type of learning of students. (ii) a learning analysis tool based on the comparison between prior knowledge and the knowledge acquired after pedagogical practice; (iii) a process for objective analysis of parameters abstracted from said analysis of an individual, thus generating metrics; (iv) a process for documenting the learning metrics of the individual through performance indicators or learning metrics; and (v) a digital database or repository of learners' profiles, learning metrics and/or academic history, with simplified access.

System and method as described above, additionally comprising a tool for building databases of learners' profiles and learning metrics.

System and method as described above, additionally comprising an auditable tool for authenticating certificates, diplomas, experiences and tokenizing learning performance metrics.

System and method as described above, additionally comprising a tool for directly encouraging and rewarding the learner for each learning achievement.

System and method as described above, additionally comprising a tool to provide suggestions for pedagogical practices specific to the individual's needs.

System and method as described above, additionally comprising a gamification tool.

System and method as described above, additionally comprising a tool for the analysis of one or more of the following layers and dimensions: assessment of the learner's reaction; assessment of learning; assessment of behavior; assessment of results; assessment of the cause-effect relationship; or combinations thereof.

System and method as described above, in which the abovementioned analysis tool weighs the metrics selected from among: number of error-free system accesses; time spent using the platform for each activity; grade given to the platform under evaluation by the learner; number of pages accessed; tools accessed; preferred activities; percentage of access completion for each contents; number of correct answers in formal tests; self-assessment grades; number of chat room participations; number of forum participations; number of e-mail or game participations; game performance; online peer evaluations in virtual group activities; number of online contacts with the teacher or tutor; quality of contributions in chat/forum/e-mail; percentage of learner/client satisfaction in surveys; reduction in the error rate in the most relevant processes; reduction of work or increase in work efficiency; value captured by the learner; grade in individual performance evaluation; grade in work unit performance evaluation; self-assessment grade as part of the performance evaluation; or combinations thereof, development of socio-emotional skills, such as resilience, self-regulation, and empathy. The combination of these metrics allows a better understanding of the student progress, the effectiveness of teaching strategies, and adapt the contents according to individual needs, making customized teaching more effective

System and method as described above, including assessment of pupil diameter.

Non-invasive method for obtaining signals and documenting learning metrics from human beings and for selecting cognitive challenges or learning tasks for the individual, comprising:
- a non-invasive stage of capturing physiological, metabolic or physical signals related to the individual's cognitive effort;
- a stage of processing said signals and documenting them and/or generating metrics or indices therefrom; and
- a feedback stage that uses said signals, metrics or indices as a criterion for selecting learning contents to be made available to the individual.

Method as described above comprising:
- a stage of capturing physiological, metabolic or physical signals related to the individual's cognitive effort, selected from: eye tracking, pupilloscopy for measuring pupil diameter and/or the speed of pupil dilation or contraction, or combinations thereof;
- a step of storing, in a database, the measured data, and processing said data to generate metrics or indices therefrom, as well as storing, in the database, said metrics or indices obtained; and
- a feedback stage that uses said signals, metrics or indices as a criterion for selecting learning contents to be made available to the individual.

In one embodiment, this method is recursive and provides the generation of measurements, metrics and/or evolution indices of the learning process. In one embodiment, this method is used both individually and collectively, that is, the step of selecting cognitive stimuli or learning tasks to be presented can be done based on the average of the metrics or indices of a subgroup of individuals, with the equivalent contents being presented to everyone in the same subgroup instead of individually.

System to assist the development of learning, comprising:
- a device for capturing physiological, metabolic or physical signals related to the individual's cognitive effort, selected from: an eye tracking device, a pupilloscope, a device that measures pupil diameter and/or pupil dilation or contraction speed, or combinations thereof, wherein said device sends signals to a processor;
- a processor for said signals that provides documentation thereof and/or the generation of metrics or indices therefrom, as well as the creation of a database in which such information is stored; and
- a feedback system that provides the selection of cognitive stimuli or learning tasks, from said database or other databases, according to pupillary measurements and/or metrics or indices derived therefrom, to be made available to the individual by a device that provides cognitive stimuli, whether visual, auditory, tactile, olfactory or combinations thereof.

System as described above, additionally comprising one or more device(s) selected from: a speaker or headset; a microphone; a screen for displaying images; a brightness or luminance meter in the eyes region; a device for generating brain images by magnetic resonance; an intracranial pressure meter; a polygraph; or combinations thereof. In this embodiment, the measurements and metrics associated with these measurements are also stored in the database.

System as described above additionally comprising a system or tool for providing suggestions for pedagogical practices specific to the needs of the individual. This tool is optionally gamified.

Equipment to aid learning development, including:
- a signal acquisition device selected from: an eye tracking device, a pupilloscope, a device that measures pupil diameter and/or pupil dilation or contraction speed, or combinations thereof;
- a processor for said signals to document them and/or to generate metrics or indices therefrom, and to create a database in which such information is stored; and
- a device for visual, auditory, tactile, olfactory feedback or combinations thereof, of cognitive stimuli or learning tasks, selected from the said database or from other databases according to pupillary measurements and/or metrics or indices derived therefrom.

In one embodiment, the system of the invention is particularly useful in the context of courses provided with digital teaching platforms, and includes: (i) a computational tool for learning analysis based on the comparison between prior knowledge and the knowledge acquired after pedagogical practice; (ii) a process for objective analysis of parameters abstracted from said analysis of an individual, thus generating metrics; (iii) a computational tool to provide suggestions for pedagogical practices specific to the individual's needs; and (iv) a process for documenting the learning metrics of the individual through performance indicators or learning evolution metrics.

In one embodiment, the system and method of the invention comprise the analysis of several layers and dimensions: assessment of the learner's reaction; assessment of learning; assessment of behavior; assessment of results; and assessment of the cause-effect relationship.

In one embodiment, the system and method of the invention integrates with AI-generated algorithms to: (i) eliminate bias by teachers or students; (ii) learn and interact in a tailored and individualized way with each student or teacher.

### Examples

The examples shown here are intended only to exemplify some of the various ways of carrying out the invention, without, however, limiting its scope.

### Example 1 - LEARNOMICS - Process for obtaining and documenting learning metrics

This example provides more details on the process for obtaining metrics and the layers and dimensions: learner's reaction assessment; learning assessment; behavior assessment; results assessment; and cause-effect relationship assessment.

The *reaction analysis* involves the learner's perception of the training/pedagogical practice platform or tool. This assessment dimension indicates the learner's adaptation to the course or learning module and involves the following items: user-friendliness of the platform (in this case, an interactive digital platform for accessing contents); ease of access to the system; learning environment; adequacy of the presentation language to the contents. The metrics for these items include, for example: number of error-free system accesses, time spent using the platform for each activity, grade given to the platform by the learner; number of pages accessed; tools accessed; percentage of access completion for each content.

The *learning analysis* involves measuring the expansion of knowledge acquired by the learner in the training/pedagogical practice in which he/she participated. This assessment dimension indicates the increase in the learner's knowledge or skills and involves the following items: online tutoring/assistance (with or without artificial intelligence); learner's performance in assessments; measurement of skill increase. The metrics for these items include, for example: number of correct answers on formal tests; self-assessment scores; number of chat room participations; number of forum participations; number of e-mail or game participations; game performance; online peer evaluations in virtual group activities; number of online contacts with the teacher or tutor.

The *behavior analysis* involves assessing the actual change in the learner's work routine. This assessment dimension indicates behavior modification and involves the following items: behavioral attendance; online tutoring/assistance (with or without artificial intelligence); learner's performance in assessments; measurement of skill increase. Metrics for these items include, for example: number of chat room participations; number of forum participations; number of e-mail participations; quality of chat/forum/e-mail contributions.

The *results analysis* involves measuring the real gains for the learner or for the organization that invested in the training program. This evaluation dimension indicates the tangible and intangible results of the actions developed in training/pedagogical practice and involves the following items: financial perspective; customer perspective; internal process perspective; innovation perspective; learning perspective; growth perspective. The metrics for these items include more relevant indicators adapted to the organizational reality and strategy, for example: percentage of learners/clients satisfaction in surveys; reduction in the error rate in the most relevant processes; reduction of work or increase in work efficiency; value captured per learner; grade in individual performance evaluation.

The *cause-effect analysis* involves analyzing processes to develop mechanisms to overcome problems and/or to understand how certain actions can influence the final product. This assessment dimension indicates the cause-effect relationships and involves the following items: relationship of the overall result with the organization's objectives; relationship of the results with the individual objectives of the learner. The metrics for these items include, for example: score in the work unit's performance evaluation; self-assessment score as part of the performance evaluation.

The system of the invention computes, processes, weighs each of the individual metrics and documents them in a database, optionally combining these metrics and weighing them to produce normalized metrics.

### Example 2 - LEARNOMICS combined with blockchain ledger/tokenization of the learning metrics

In one embodiment, the system and method of the invention further comprises a blockchain ledger/tokenization tool for learning metrics. This peculiar feature of this embodiment of the invention solves several technical problems and provides numerous advantages. On the one hand, the problem of obtaining and documenting learning metrics is solved with the system of the invention, as exemplified in Example 1.

Another problem solved by the invention is the reliability of the metrics and their documentation, given the indelible form of documentation of evaluations and metrics provided by tokenization.

In one embodiment of the invention, the recording of learning metrics (or biophysical signals from the learner, as further detailed in Example 4 below) is carried out with an IoT (Internet of Things) device that provides the automatic capture of learning-related data in real or near real-time. In this context, one of the present inventors co-invented an loT/ software with these characteristics, for the integration of various sensors, systems and processes for recording data directly and automatically in blockchain, being the object of the Patent Application BR102022003697-7, published on 09/05/2023, entitled "Dispositivo e Sistema para Medição de Grandezas, Tratamento de Dados e Integração com Sistema de Emissão de Ativos Digitais ou Tokens" [Device and System for Measuring Quantities, Data Processing and Integration with Digital Asset or Token Issuance System] and incorporated herein by reference.

Said device was designed to capture data from various sources, such as sensors and monitoring equipment, in order to document metrics directly and without human interference. Therefore, said device is a system for measuring quantities, processing data and integrating with a system for issuing digital assets or tokens in blockchain-type networks. The device may be in the form of an loT device comprising: one or more devices or systems for measuring entities/quantities, providing learning metrics; and a process for recording (in a distributed digital system or blockchain) authenticated metrics. In this case, the device comprises a processor, data storage memory, and embedded software to process data from the said measured quantities and transform them into information to be stored on the blockchain through the process of tokenizing the quantity of the measured quantity. In another embodiment, the solution is implemented as software, which can be embedded in a preexisting measuring device, in order to perform calculations, obtain the quantities read from the medium from the device's primary memory and compute the learning metrics.

Another relevant aspect in this embodiment is the blockchain platform used, since within the scope of the present invention most of them are technically unfeasible. In this implementation, a blockchain ledger platform with XRP Ledger technology was used, specifically chosen for its robustness and high reliability, scalability, speed, and energy efficiency. The XRP Ledger's attributes provide the platform with great transparency, immutability, and documentation of related data, making it tamper-proof. In the present embodiment, learning metrics are recorded as transactions in the XRP Ledger, whose consensus algorithm and cryptographic protocols ensure the integrity and security of data stored on the blockchain. This ensures that information related to learning metrics remains immutable and resistant to possible fraud or modification attempts.

Important characteristics of the XRP Ledger technology, which were used as criteria for its selection in the implementation of the present invention, include: its extreme speed, very low energy consumption and high scalability. Table 1 shows these aspects and shows that XRP Ledger technology stands out when compared to other known technologies for recording data on the blockchain. Its speed, in particular, is compatible with the object of the invention, which is the generation of learning metrics in real or near real-time, making the concept of the invention technically viable.

**Table 1 - Attributes of different blockchain data recording technologies.**

| **Attribute\Technology** | **Ethereum** | **Bitcoin** | **XRP Ledger** |
|---|---|---|---|
| Time per record/transaction | 20 min | 2 h | 3s |
| Cost per record/transaction | US$ 15 | US$ 25 | US$ 0.0004 |
| Scalability (transactions/second) | 16 tps | 32 tps | 1500 tps |
| Energy consumption per transaction | 4.5 kWh | 707 kWh | 0.0079 kWh |

The technical aspect of enabling the documentation of data in real time, which results from the combination of the characteristics of the XRP Ledger technology with the use of the IoT device described above, due to its extremely high speed, allows the documentation of learning metrics continuously or *quasi* continuously. This in turn enables, for the first time, a feedback system in which learning data obtained from the learner is documented in blockchain and can be used as a contents selection criterion, according to documented metrics. The solution also provides better process monitoring, encourages greater accountability, and improves informed decision-making processes, facilitating the rapid identification of areas for further improvement.

One aspect of the invention is to provide a direct economic rationale for the activity of education, by enabling the reward of individual dedication or the investment of third parties in the education process. This aspect is made possible through the invention's tokenization system, in which the platform integrates the educational track with a token system based on performance.

Those skilled in the art will know that various asset tokenization approaches can be utilized. In the present invention, preference is made to the tokenization process, which is the subject of pending patent application BR102022003708-6, which has one of the inventors in common with the present inventors. Said patent application, herein incorporated by reference, discloses an integrated system and method for issuing digital assets or tokens backed by authenticated metrics. The system is particularly useful for the measurement, documentation, authentication and tokenization, in blockchain platforms or technologies, distributed digital ledger, of metrics related to sustainable or environmental conservation attitudes and/or projects, as it occurs in the present invention. Said system is adapted in the present invention to comprise: one or more learning metrics authentication devices; and a distributed digital ledger process of the authenticated metrics.

Optionally, the system of the invention (LEARNOMICS) and/or the tokenization system described in this example, additionally comprises a device for verifying the authenticity of the hardware and software that processes the learning metrics and that makes up the system of the invention.

The tokenization system of the metrics provided by the system (LEARNOMICS) of the invention is particularly useful for the online documentation of authenticated metrics that serve as a collateral for the digital assets documented by the system itself, being a useful tool to boost investments in projects involving crypto assets, in addition to providing real, online and publicly auditable collateral for digital assets. In this embodiment of the present invention, said system additionally provides a peculiar way of documenting learning metrics.

Referring to Fig. 1, which shows a schematic representation of this embodiment of the invention, the system of the invention (LEARNOMICS) comprises a tool for tokenizing learning metrics. In 11, the platform is shown as exemplified in Example 1, producing the metrics indicated in 12; in 13, the token generation platform (TGOs) and certificates (GBKs) is shown; in 14, a centralized or decentralized crypto asset exchange is shown, which can operate commercially in conjunction with the crypto asset market (for example, Ripple's XRPs) or traders 16; in 15, one or more companies or people are shown that support the learning process through the purchase of crypto assets (TGOs) and exchange for certificates (GBKs).

In addition to providing indelible documentation of the metrics generated and thus providing a reliable tool for online assessment of learning processes, the tokenization exemplified above also provides an alternative for financing and rewarding learning efforts, whether in the form of certificates or direct financial incentives to the learner.

In an alternative embodiment, the system and method of the invention additionally comprise a tool for directly encouraging and rewarding the learner for each learning achievement. This reward can be given in the form of tokens directly, with economic value and usable in exchanges, in the form of individualized digital certificates such as NFTs (non-fungible tokens), or combinations thereof.

In an alternative embodiment, the system and method of the invention further comprise a tool to provide suggestions for pedagogical practices specific to the individual's needs. Said tool is optionally gamified, thus providing engagement, stimuli, documentation, and authentication of learning information.

### Example 3 - LEARNOMICS with Digital Learning Wallet

In one embodiment, the system and method of the invention further comprise a database or digital learning repository or Digital Learning Wallet, which integrates all of the individual's academic information in an easily accessible, secure, and auditable location.

This implementation comprises an authenticated documentation tool for learning metrics and academic history, serving as a digital repository containing this data, with simplified access.

Learning occurs outside and inside the classroom. People learn from different sources and ways and undergo different educational experiences throughout their lives. The conventional academic record does not reflect the complex learning process, as it focuses only on the student's formal education. Non-formal activities are essential for learning, but they are not reflected, recorded or valued throughout life, not even in the school curriculum.

The invention is particularly useful for providing a consolidated record of all educational activities, both official and unofficial, throughout an individual's life (lifelong learning). In this context, the invention provides a self-management tool for recording and measuring individuals' lifelong learning.

The invention also provides a corporate or government tool that concentrates, values, and rewards this information. Students, students' families, groups of students or professionals, companies, government agencies, and philanthropists all benefit from it.

The aspect of the invention of building a planetary database on learning processes provides a tool for organizing the educational journey, with details about the learning process for each concept/practice, as well as diplomas, certificates, and other experiences. Everything is centralized in a Digital Learning Wallet that is continuously updated according to learning activities.

The tool is also useful for mapping the market, getting to know the professionals and their authenticated skills (not merely the declared ones), providing more quality to the job candidate selection process, for example.

The storage and easy availability of the Online Teaching + Learning History results from the digital file saved in the cloud, containing a standardized system for the storage of teaching and learning data that are tokenized. In addition to capturing and storing personal data and teaching + learning data, the invention is useful for other issues such as the storage of messages exchanged between education professionals, or between educational institutions or the corporate world, and people can manage reminders for studies, exams, tests, and organize other appointments.

Information about eternal students/professionals can be accessed from anywhere (schools, companies), a concept aligned with the Concept of Digital Citizen / Digital Professional / Global Citizen / Digital Student / Global Student, who has access and control over their educational and learning journey, as well as use of the reward system described above.

### Example 4 - Method for obtaining documentation of signals and metrics of cognitive effort and/or learning

This example describes a non-invasive method for obtaining signals and documenting learning metrics from human beings and for selecting cognitive challenges or learning tasks for the individual.

In this embodiment, said method comprises: - a stage of capturing physiological, metabolic or physical signals related to the individual's cognitive effort, selected from: eye tracking, pupilloscopy for measuring pupil diameter and/or the speed of pupil dilation or contraction, or combinations thereof; - a step of storing, in a database, the measured data, and processing said data to generate metrics or indices therefrom, as well as storing, in the database, said metrics or indices obtained; and - a feedback stage that uses said signals, metrics or indices as a criterion for selecting learning contents to be made available to the individual.

The method of this embodiment of the invention is depicted in conjunction with Fig. 2. Fig. 2 shows a schematic representation of a human eye, with the following indicated: in A, the eye in a normal state; in B, an eye with a dilated pupil; in C, an eye is shown in a schematic side view of the eye shown in A, with the following highlighted: 21 the pupil; 22 a beam of light; 23 the retina; and 24 the optic nerve; in D, an eye is shown in schematic side view of the eye shown in B, i.e., with the pupil dilated. Fig. 3 shows a schematic representation of a pupil examination being performed with a portable pupillometer, which can be attached to a monitor or screen where the contents to which the cognitive load is related are displayed.

In one embodiment, this method is recursive and provides the generation of measurements, metrics and/or evolution indices of the learning process. In this embodiment, the recording of learning metrics (or biophysical signals from the learner) is preferably done with an IoT device that provides the automatic capture of data related to real or near real-time learning from the integration of one or more biophysical data sensors, such as an eye tracking, pupilloscopy or others, and their data being automatically recorded in blockchain, as described in BR102022003697-7, herein incorporated by reference.

In one embodiment, this method is used both individually and collectively, that is, the step of selecting cognitive stimuli or learning tasks to be presented can be done based on the average of the metrics or indices of a subgroup of individuals, with the equivalent contents being presented to everyone in the same subgroup instead of individually.

In one embodiment, the method includes the evaluation of specific layers and dimensions: assessment of the learner's reaction; assessment of learning; assessment of behavior; assessment of results; and assessment of the cause-effect relationship. These analyses are used as criteria for converting the measurements made into metrics or indices.

The method of such an embodiment of the invention computes, processes, weighs each of the individual metrics and documents them in a database, optionally combining these metrics and weighing them to produce normalized metrics.

In one embodiment, the method is recursive and provides the generation of measurements, metrics and/or evolution indices of the learning process. In one embodiment, this method is used both individually and collectively, that is, the step of selecting cognitive stimuli or learning tasks to be presented can be done based on the average of the metrics or indices of a subgroup of individuals, with the equivalent contents being presented to everyone in the same subgroup instead of individually.

### Example 5 - LEARNOMICS, a system for obtaining documentation of signals and metrics of cognitive effort and/or learning

This example describes a system embodiment for obtaining and documenting signals and metrics of cognitive effort and/or learning. Said system comprises: - a device for capturing physiological, metabolic or physical signals related to the individual's cognitive effort, selected from: an eye tracking device, a pupilloscope, a device that measures pupil diameter and/or pupil dilation or contraction speed, or combinations thereof, wherein said device sends signals to a processor; - a processor for said signals that provides documentation thereof and/or the generation of metrics or indices therefrom, as well as the creation of a database in which such information is stored; and - a feedback system that provides the selection of cognitive stimuli or learning tasks, from said database or other databases, according to pupillary measurements and/or metrics or indices derived therefrom, to be made available to the individual by a device that provides cognitive stimuli, whether visual, auditory, tactile, olfactory or combinations thereof.

The system of this embodiment of the invention can be described in conjunction with Fig. 3, in which a schematic diagram representation of the system is shown, in which a pupillometry examination step results in the indication of the pupil diameter and/or the rate of change of the pupil diameter in response to a cognitive stimulus. A system for obtaining signals and documenting human learning metrics includes: a pupillometer 31 generating measurements of pupil diameter and/or the rate of change of pupil diameter 32a, 32b in response to a cognitive stimulus, which may be visual, auditory, tactile, olfactory or combinations thereof; the measurements performed by the pupillometer are stored in a database 33. Said database stores the actual measurements and/or metrics or indices derived from these measurements, as well as the cognitive stimulus presented to the individual, to provide a subsequent correlation between the stimulus and the measurements, metrics and indices. In one embodiment, the system is recursive and provides selection of cognitive stimuli or learning tasks according to pupillary measurements and/or metrics or indices derived therefrom.

In one embodiment, said system additionally comprises other devices selected from: a speaker or headset; a microphone; a screen for displaying images; a brightness or luminance meter in the eyes region; a device for generating brain images by magnetic resonance; an intracranial pressure meter; a polygraph; or combinations thereof. In this embodiment, the measurements and metrics associated with these measurements are also stored in the database.

In one embodiment, the system of the invention further comprises a system or tool for providing suggestions for pedagogical practices specific to the individual's needs. This tool is optionally gamified.

Those skilled in the art will appreciate the knowledge presented herein and will be able to reproduce the invention in the presented embodiments and in other variants and alternatives within the scope of the following claims.

## Claims

1. System and method for obtaining and documenting learning and learning development metrics of individuals, **characterized in that** it comprises one or more of the following elements: (i) a learning analysis tool based on the comparison between prior knowledge and the knowledge acquired after pedagogical practice; (ii) a process for objective analysis of parameters abstracted from said analysis of an individual, thus generating metrics; (iii) a process for documenting the learning metrics of the individual through performance indicators or learning metrics; and (iv) a digital database or repository of learners' profiles, learning metrics and/or academic history, with simplified access.

2. System and method according to claim 1, **characterized in that** it additionally comprises a tool for constructing databases of learners' profiles and learning metrics.

3. System and method according to claim 1 or 2, **characterized in that** it additionally comprises a tool for tokenizing learning performance metrics.

4. System and method according to claim 3, **characterized in that** it additionally comprises a tool for directly encouraging and rewarding the learner for each learning achievement.

5. System and method according to any one of claims 1 to 4, **characterized in that** it additionally comprises a tool to provide suggestions for pedagogical practices specific to the individual's needs.

6. System and method according to any one of claims 1 to 5, **characterized in that** it additionally comprises a gamification tool.

7. System and method according to any one of claims 1 to 6, **characterized in that** it additionally comprises a tool for analyzing one or more of the following layers and dimensions: assessment of the learner's reaction; assessment of learning; assessment of behavior; assessment of results; assessment of the cause-effect relationship; or combinations thereof.

8. System and method according to claim 7, **characterized in that** said analysis tool weighs the metrics selected from among: number of error-free system accesses; time spent using the platform for each activity; grade given to the platform under evaluation by the learner; number of pages accessed; tools accessed; percentage of access completion for each contents; number of correct answers in formal tests; self-assessment grades; number of chat room participations; number of forum participations; number of e-mail or game participations; game performance; online peer evaluations in virtual group activities; number of online contacts with the teacher or tutor; quality of contributions in chat/forum/e-mail; percentage of learner/client satisfaction in surveys; reduction in the error rate in the most relevant processes; reduction of work or increase in work efficiency; value captured by the learner; grade in individual performance evaluation; grade in work unit performance evaluation; self-assessment grade as part of the performance evaluation; or combinations thereof.

9. Non-invasive method for obtaining signals and documenting learning metrics from human beings and for selecting cognitive challenges or learning tasks for the individual, **characterized in that** it comprises:
- a non-invasive stage of capturing physiological, metabolic or physical signals related to the individual's cognitive effort;
- a stage of processing said signals and documenting them and/or generating metrics or indices therefrom; and
- a feedback stage that uses said signals, metrics or indices as a criterion for selecting learning contents to be made available to the individual.

10. Method according to claim 9, **characterized in that** it comprises a recursive step to provide the generation of measurements, metrics and/or evolution indices of the learning process.

11. Method according to any one of claims 9 to 10, **characterized in that** the step of selecting cognitive stimuli or learning tasks to be presented is carried out based on the average of the metrics or indices of a subgroup of individuals, with the equivalent contents being presented to everyone in the same subgroup.

12. System for obtaining signals and documenting learning metrics from human beings and for selecting cognitive challenges or learning tasks for the individual, **characterized in that** it comprises:
- a device for capturing physiological, metabolic or physical signals related to the individual's cognitive effort, selected from: an eye tracking device, a pupilloscope, a device that measures pupil diameter and/or pupil dilation or contraction speed, or combinations thereof, wherein said device sends signals to a processor;
- a processor for said signals that provides documentation thereof and/or the generation of metrics or indices therefrom, as well as the creation of a database in which such information is stored; and
- a feedback system that provides the selection of cognitive stimuli or learning tasks, from said database or other databases, according to pupillary measurements and/or metrics or indices derived therefrom, to be made available to the individual by a device that provides cognitive stimuli, whether visual, auditory, tactile, olfactory or combinations thereof.

13. System according to claim 12, **characterized in that** it additionally comprises one or more devices selected from: a speaker or headset; a microphone; a screen for displaying images; a brightness or luminance meter in the eyes region; a device for generating brain images by magnetic resonance; an intracranial pressure meter; a polygraph; or combinations thereof.

14. System according to any one of claims 12 to 13, **characterized in that** it additionally comprises a tool for tokenizing learning performance metrics.

15. Equipment to assist the development of learning, **characterized in that** it comprises:
- a signal acquisition device selected from: an eye tracking device, a pupilloscope, a device that measures pupil diameter and/or pupil dilation or contraction speed, or combinations thereof;
- a processor for said signals to document them and/or to generate metrics or indices therefrom, and to create a database in which such information is stored; and
- a device for visual, auditory, tactile, olfactory feedback or combinations thereof, of cognitive stimuli or learning tasks, selected from the said database or from other databases according to pupillary measurements and/or metrics or indices derived therefrom.
